# FASCICULE DE BREVET EUROPEEN

(11) **EP 2 766 334 B1**
(45) Date de publication et mention de la délivrance du brevet: **08.01.2020**
(21) Numéro de dépôt: 12790648.5
(22) Date de dépôt: 09.10.2012
(51) Int. Cl.: C07C 47/225, A61Q 13/00, A61K 8/33

(54) **NOUVEAUX ALDEHYDES CYCLOALCANIQUES, LEUR PROCEDE DE PREPARATION AINSI QUE LEUR UTILISATION EN PARFUMERIE**
NEUE CYCLOALKANALDEHYDE, VERFAHREN ZU IHRER HERSTELLUNG UND VERWENDUNG DAVON IN DER PARFÜMINDUSTRIE
NOVEL CYCLOALKANE ALDEHYDES, METHOD FOR PREPARING SAME, AND USE THEREOF IN THE PERFUME INDUSTRY

(30) Priorité: 11.10.2011 FR 1159180
(43) Date de publication de la demande: 20.08.2014
(73) Titulaire: V. Mane Fils, 06620 Le Bar-sur-Loup (FR)
(72) Inventeur: MURATORE, Agnès, 06740 Chateauneuf (FR); PLESSIS, Caroline, 06740 Chateauneuf (FR); CHANOT, Jean-Jacques, 06530 Speracedes (FR)
(74) Mandataire: Murgitroyd & Company
(86) Numéro de dépôt international: PCT/IB2012/055447
(87) Numéro de publication internationale: WO 2013/054253

(56) Documents cités:
- EP-A1- 1 930 317
- WO-A1-2013/034657
- WO-A2-2012/156649
- FR-A- 1 367 793
- FR-A5- 2 038 865
- Lutz F. Tietze ET AL: "Inter- and intramolecularhetero diels-alder reactions, XXI. Intramolecularhetero diels-alder reaction of alkylidene-1,3-dicarbonyl compounds. Experimental evidence for an asymmetric transition state", Chemische Berichte, vol. 121, no. 3, 1 March 1988 (1988-03-01) , pages 499-506, XP055026659, ISSN: 0009-2940, DOI: 10.1002/cber.19881210318

## Description

La présente invention concerne de nouveaux composés aldéhydes cycloalcaniques, leur procédé de préparation et leur utilisation dans l'industrie de la chimie, et en particulier en parfumerie, cosmétique, et dans l'industrie des détergents, lesdits composés présentant une fragrance particulière.

L'industrie des parfums et arômes est toujours à la recherche de composés organoleptiques nouveaux, présentant un pouvoir olfactif intense, tout en ayant des coûts de production les plus limités possibles. Certains types de composés organoleptiques sont plus difficiles à obtenir que d'autres, comme par exemple les composés ayant des notes marines et/ou ozoniques.

Parmi les composés décrits dans l'art antérieur comme ayant des notes marines et/ou ozoniques, on trouve, parmi les plus utilisés, les dérivés de type benzodioxepinone (Helvetica Chimica Acta, 2007, 90, 1245-1265) comme la Calone®, ou encore comme l'Azurone® (Givaudan) ou la 7-(3-méthylbutyl)benzo[B][1,4]dioxepin-3-one (brevet EP1136481).

Certains composés de la famille des aldéhydes sont également connus pour présenter ce type de notes marines et ozoniques. Citons par exemple, le Melozone® (hexahydro-1-carboxaldéhyde-4,7-méthanoindane, brevet DE19817042) qui associe en plus des notes marines et ozoniques, des notes aldéhydées. Ou encore, le Geraldéhyde® (5,9-diméthyl-4,8-decadiènal, brevets FR13677165 et FR2005165) qui associe des notes citronnées, ou encore la Floralozone® (3-(4-éthylphényl)-2,2-diméthylpropanal, Perfumer & Flavorist, 2009, 34, 18-19) qui associe des notes florales.

Cependant, les composés de l'art antérieur possédant une note marine ou ozonique originale, ont comme principal inconvénient leur coût de production élevé, lié notamment au nombre d'étapes de synthèse, ou encore au coût des matières premières. A titre d'illustration, le procédé de synthèse du 7-méthyl-3,4-dihydro-2H-1,5-benzodioxepin-3-one décrit dans le brevet US 3517031, comporte trois étapes, ce qui est conséquent. En outre, le substrat de départ, le pyrocatéchol, est une matière première au coût significativement élevé. De même, le procédé de synthèse du 3-méthyl-6-(2,2,3-triméthylcyclopentyl)-hexanal (décrit comme marin, ozonique dans la demande de brevet EP1930317) comporte cinq étapes de synthèse à partir de l'aldéhyde campholénique.

Afin de surmonter les inconvénients de l'art antérieur, la Demanderesse, a de manière surprenante découvert que des aldéhydes cycloalcaniques répondant à la formule générale (I) suivante, 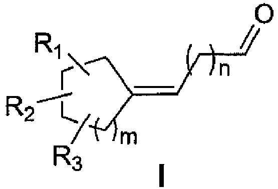 présentaient des notes marines et/ou ozoniques très intenses, et étaient obtenus à l'aide d'un procédé simple, mis en œuvre en 2 étapes à partir de composés de départ simples et peu coûteux.

Certains aldéhydes cycloalcaniques répondant à la formule générale (I) précédente, ont été divulgués dans l'art antérieur mais jamais comme présentant des notes marines et/ou ozoniques. Il s'agit des composés suivants :
- le 6-cycloheptylidènehexanal
- le 4-(4-méthylcyclohexylidène)-butanal
- le 4-(4-tert-butylcyclohexylidène)-butanal
- le 4-(3,3,5-triméthylcyclohexylidène)-butanal
Le composé 6-cycloheptylidènehexanal est décrit dans un article de Tietze et al. (Chem. Ber. 121, 499-506, 1988) comme simple intermédiaire réactionnel dépourvu d'une quelconque application industrielle directe qu'elle soit de nature organoleptique ou non. Quant aux 3 autres composés divulgués dans l'art antérieur, tous sont décrits dans un brevet de Naarden (FR2038865). Dans ce brevet, le 4-(4-méthylcyclohexylidène)-butanal est décrit comme présentant des notes d'herbe fraîche rappelant celle du muguet ; le 4-(4-tert-butylcyclohexylidène)-butanal est décrit comme ayant une odeur florale relevée ; et le 4-(3,3,5-triméthylcyclohexylidène)-butanal est décrit comme ayant une odeur florale boisée rappelant fortement celle du muguet.
Par conséquent, aucun des composés répondant à la formule générale (I), et en particulier aucun des 4 composés aldéhydes ci-dessus, n'est décrit dans l'art antérieur comme présentant des notes marines et/ou ozoniques.
La présente invention a donc pour premier objet un composé de formule générale (I) suivante : 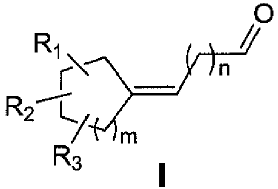 dans laquelle :
- R1, R2 et R3 représentent chacun indépendamment un atome d'hydrogène ou un groupement alkyle en C1-C5 saturé ou insaturé, ramifié ou non ramifié ;
- m est un nombre entier compris entre 1 et 4 ;
- n est un nombre entier compris entre 2 et 4 ;
caractérisé en ce que le cycle est saturé et comprend de 5 à 8 carbones, que le nombre total de carbones du cycle et des radicaux R1, R2 et R3 est compris entre 7 et 11
et étant entendu que ledit composé de formule (I) n'est pas :
- le 6-cycloheptylidènehexanal
- le 4-(4-méthylcyclohexylidène)-butanal
- le 4-(4-tert-butylcyclohexylidène)-butanal
- le 4-(3,3,5-triméthylcyclohexylidène)-butanal
- le 4-(3,3-diméthylcyclopentylidène)-butanal
- le 4-(3-éthyl-3-méthylcyclopentylidène)-butanal.

Ces composés présentent une note marine, ozonique, aqueuse, voire fruitée (côté melon, pastèque) ou florale, très puissante et diffusive, ainsi qu'une rémanence remarquable. De plus, ils sont obtenus grâce à un procédé mise en œuvre en 2 étapes simples.

Par le terme « alkyle en C1-C5 » au sens de la présente invention, on entend tout radical monovalent dérivé d'une chaîne carbonée saturée ou insaturée, linéaire ou ramifiée contenant 1 à 5 atomes de carbones. De manière préférée, les alkyles en C1-C5 sont choisis parmi les groupes méthyle, éthyle, *n-*propyle, *i*-propyle, *n*-butyle, *i*-butyle, *t*-butyle, et pentyle.

Dans un premier mode de réalisation, la présente invention concerne les composés de formule générale (I) dans laquelle m est égal à 1, c'est-à-dire que le cycle est un cyclopentane.

Dans un second mode de réalisation, la présente invention concerne les composés de formule générale (I) dans laquelle n est égal à 4. Un troisième mode de réalisation consiste à ce que n est égal à 2.

Dans un mode de réalisation préféré, les composés selon l'invention sont choisis parmi le 5-(2,4,4-triméthylcyclopentylidène)-pentanal, le 6-(2,4,4-triméthylcyclopentylidène)-hexanal, le 6-(2-méthylcyclohexylidène)-hexanal, le 6-(4-méthylcyclohexylidène)-hexanal, le 6-(4-tert-butylcyclohexylidène)-hexanal, le 6-(4-tert-amylcyclohexylidène)-hexanal, le 6-cyclooctylidènehexanal, le 6-(3,3-diméthylcyclohexylidène)hexanal, le 4-(2,4,4-triméthyl cyclopentylidène)butanal, le 4-(2-pentylcyclopentylidène)-butanal, le 4-(3,3-diméthyl cyclohexylidène)-butanal, le 5-(4,4-diéthylcyclohexylidène)-pentanal et le 5-cycloheptylidènepentanal.

Plus particulièrement, l'invention concerne les composés de formule (I) représentés dans le tableau 1 ci-dessous.

**Tableau 1**

| **Exemple** | **Structure** | **Description olfactive (à 5% massique dans le dipropylène glycol, DPG)** |
|---|---|---|
| Exemple 1 | 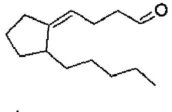 | Algues séchées, minéral, aldéhydé, gras, hespéridé (peau d'orange) |
| Exemple 2 | 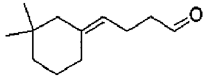 | Marin, aqueux, concombre, vert (pomme), épicé (cardamome), aldéhydé, fleuri |
| Exemple 3 | 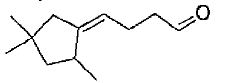 | Aqueux, concombre, melon, ozonique, vert, fleuri |
| Exemple 4 | 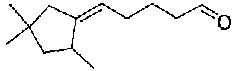 | Ozonique, fer chaud, vert, gras, aldéhydé |
| Exemple 5 | 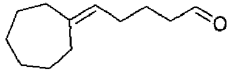 | Aqueux, concombre, aldéhydé, plastique, animal, cuiré, gustatif (amandé, coumarine) |
| Exemple 6 | 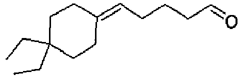 | Ozonique, côté algue séchée, salé, fleuri, gras, vert |
| Exemple 7 | 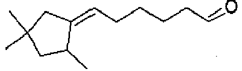 | Marin, aqueux, ozonique, vert, aldéhydé, coriandre |
| Exemple 8 | 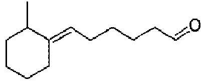 | Aldéhydé, chloré, poudré, chocolat, animal |
| Exemple 9 | 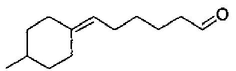 | Aqueux, chloré, aldéhydé, gustatif (amande, coumariné), poussiéreux |
| Exemple 10 | 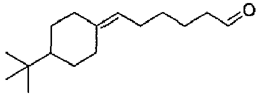 | Ozonique, aldéhydé, gras, poudré, chocolat |
| Exemple 11 | 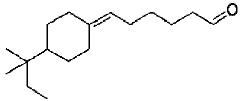 | Marin, aqueux, minéral, algue |
| Exemple 12 | 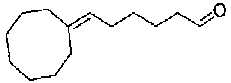 | Aqueux, chocolat, aldéhydé, rance, gustatif, boisé |
| Exemple 13 | 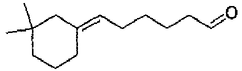 | Ozonique, aqueux, marin, vert, aldéhydé, anisé, fleuri |

L'invention comprend tous les énantiomères et diastéréoisomères des composés de formule (I), seuls ou en mélange. En particulier, l'invention comprend les composés représentés par la formule générale (I) sous forme de mélanges d'énantiomères en proportions variables, notamment les mélanges racémiques. L'élaboration des mélanges d'énantiomères ou de formes pures est réalisée par des méthodes connues de l'homme de l'art en utilisant, par exemple, des produits de départ optiquement enrichis ou optiquement purs.

Les composés de formule (I) possèdent l'avantage d'être accessibles par une préparation fiable et peu coûteuse, puisque réalisable en 2 étapes à partir de composés de départ à faible coût.

Un second objet de la présente invention concerne une composition comprenant au moins un composé de formule générale (I) à l'exception des composés 6-cycloheptylidènehexanal, 4-(4-méthylcyclohexylidène)-butanal, 4-(4-tert-butylcyclohexylidène)-butanal et 4-(3,3,5-triméthylcyclohexylidène)-butanal, sous forme d'isomère ou d'un mélange d'isomères, d'un énantiomère ou d'un mélange d'énantiomères, ou d'un mélange racémique, ou d'un diastéréoisomère ou d'un mélange de diastéréisomères.

Selon un mode de réalisation, la composition est caractérisée en ce qu'elle comprend en outre au moins une autre substance odorante.

La quantité efficace des composés de formule (I) selon l'invention incorporée dans la composition variera selon la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier de 0,1 à 50% en poids, notamment de 0,1 à 30% en poids.

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade comprenant au moins un composé de formule (I), ou au moins une composition comprenant au moins un composé de formule (I) étant entendu que les composés de formule (I) ne sont pas les composés 6-cycloheptylidènehexanal, 4-(4-méthylcyclohexylidène)-butanal, 4-(4-tert-butylcyclohexylidène)-butanal et 4-(3,3,5-triméthylcyclohexylidène)-butanal.

L'invention concerne encore un produit d'entretien, notamment assouplissant, détergent, lessive, désodorisant d'ambiance, comprenant au moins un composé de formule (I) ou au moins une composition comprenant au moins un composé de formule (I) étant entendu que les composés de formule (I) ne sont pas les composés 6-cycloheptylidènehexanal, 4-(4-méthylcyclohexylidène)-butanal, 4-(4-tert-butylcyclohexylidène)-butanal et 4-(3,3,5-triméthylcyclohexylidène)-butanal.

Les composés selon l'invention peuvent être utilisés, seuls ou en combinaison, tels quels ou être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

Un troisième objet de la présente invention concerne un procédé de préparation d'un composé de formule (I) dans lequel une cycloalcanone et un ylure de phosphore sont soumis à une réaction de Wittig, suivie d'une étape de réduction et/ou d'oxydation selon l'ylure utilisé précédemment.

Le procédé selon l'invention comprend notamment les étapes de :
i) addition d'un ylure de phosphore de formule (III) sur une cycloalcanone de formule (II), selon une réaction de Wittig : 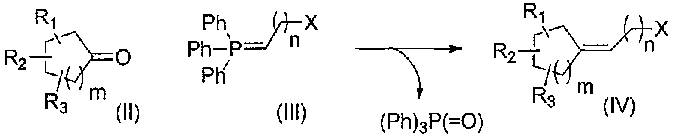 R₁, R₂, R₃, n et m étant tels que définis ci-dessus et X représentant une fonction nitrile, ester carboxylique ou alcool ; et
ii) conversion de la fonction X du composé (IV) obtenu en aldéhyde, par réduction et/ou oxydation, ce par quoi on obtient le composé de formule (I).

Selon un mode de réalisation, le procédé selon l'invention comprend au moins les étapes suivantes :
- une étape d'addition d'un ylure de phosphore sur une cycloalcanone pour l'obtention d'un composé intermédiaire, ledit ylure de phosphore comprenant :
   ∘ au total 3 carbones et une fonction nitrile, en vue de l'obtention d'un composé de formule I pour lequel la chaîne latérale comprend au total 4 carbones (n = 2), ou
   ∘ au total 5 carbones et une fonction ester en vue de l'obtention d'un composé de formule I pour lequel la chaîne latérale comprend au total 5 carbones (n = 3), ou
   ∘ au total 6 carbones et une fonction alcool en vue de l'obtention d'un composé de formule I pour lequel la chaîne latérale comprend au total 6 carbones (n = 4),
- suivie d'une étape ou de deux étapes consistant à convertir ledit composé intermédiaire en aldéhyde correspondant.
Lorsque le composé intermédiaire est un nitrile (n = 2), l'étape permettant d'obtenir l'aldéhyde I de la présente invention correspondant consiste en une réduction à l'aide d'hydrure de diisobutylaluminium, selon des protocoles connus de l'homme de l'art.
Lorsque le composé intermédiaire est un ester (n = 3), une étape de saponification permet d'obtenir, selon des modes opératoires connus de l'homme de l'art, l'alcool correspondant.
Lorsque le composé intermédiaire est un alcool, (n = 3 ou 4), l'étape permettant d'obtenir l'aldéhyde I de la présente invention consiste en une oxydation, selon des modes opératoires connus de l'homme de l'art. Par exemple, on peut noter l'utilisation de TEMPO et d'hypochlorite de sodium (Oxydation d'Anelli) ou l'oxydation de Swern ou celle de Dess-Martin, ou l'utilisation de métaux de transition supportés (charbon, notamment) sous atmosphère d'oxygène ou d'air.

L'invention a pour dernier objet l'utilisation d'au moins un composé de formule (I) selon l'invention à l'exception des composés 6-cycloheptylidènehexanal, 4-(4-méthylcyclohexylidène)-butanal, 4-(4-tert-butylcyclohexylidène)-butanal et 4-(3,3,5-triméthylcyclohexylidène)-butanal comme agent ou composé fragrant, comme agent de masquage d'odeur ou comme agent de neutralisation d'odeur. Les termes «fragrant», «odorant» sont utilisés ici de manière interchangeable pour désigner tout composé organoleptique stimulant de façon plaisante l'odorat. Par le terme «agent de masquage» ou «masquant» on entend réduire ou éliminer la perception d'une mauvaise odeur générée par une ou plusieurs molécules entrant dans la composition d'un produit.

En outre, ledit composé peut être utilisé seul ou en combinaison avec au moins un autre ingrédient aromatisant ou parfumant, et/ou au moins un solvant, et/ou au moins un adjuvant. Le ou les agents odorants supplémentaires peuvent être des composés de formule (I) ou d'autres agents odorants connus de l'homme du métier qui sera à même de choisir en fonction de l'effet recherché.

De manière générale, les composés selon l'invention seront utilisés dans le domaine de la parfumerie. On entend par « parfumerie » non seulement la parfumerie au sens habituel du terme, mais également les autres domaines dans lesquels l'odeur des produits est importante. Il peut s'agir de compositions de parfumerie au sens habituel du terme, telles que bases et concentrés parfumant, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, anti-transpirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, et produits similaires.

Un mode de réalisation particulier de l'invention réside en l'utilisation d'un composé de formule (I) à l'exception des composés 6-cycloheptylidènehexanal, 4-(4-méthylcyclohexylidène)-butanal, 4-(4-tert-butylcyclohexylidène)-butanal et 4-(3,3,5-triméthylcyclohexylidène)-butanal pour conférer, modifier ou renforcer les propriétés organoleptiques d'une substance, d'une composition ou d'un article.

On entend par «propriétés organoleptiques» toute propriété susceptible de modifier, améliorer ou renforcer la perception organoleptique d'une substance, d'une composition, d'un article par un utilisateur. Ainsi, à titre d'exemple préférentiel, l'agent organoleptique selon l'invention peut consister en un agent parfumant susceptible de conférer, modifier, améliorer ou renforcer la perception olfactive d'une substance, d'une composition ou d'un article.

Le principe général de l'invention repose sur la préparation et l'utilisation en parfumerie des composés de formule I décrits précédemment. Les exemples suivants illustrent une manière particulière de préparer les composés de l'invention, ainsi que le profil olfactif de chacun des composés exemplifiés (voir tableau 1 pour les descriptions olfactives). Ces exemples ne sont donnés que dans un but d'illustration et ne doivent pas être compris comme limitant la portée générale de l'invention.

### Exemple 1: Préparation du 4-(2-pentylcyclopentylidène)-butanal

Dans un ballon, on place un équivalent de 4-chlorobutyronitrile et un équivalent de triphénylphosphine dans le dibutyle éther à 140 °C. Après 20 heures d'agitation dans ces conditions, et une fois revenu à température ambiante, le chlorure de 3-(cyanopropyl)-triphénylphosphonium formé est filtré sur fritté, rincé au méthyle tert-butyle éther (MTBE) puis séché sous vide.

On place dans un ballon 1 équivalent de chlorure de 3-(cyanopropyl)-triphénylphosphonium et 1,2 équivalents de tertio-butylate de potassium dans du THF sec. On agite cette suspension à 60 °C pendant 2 heures. Puis, on ajoute 1 équivalent de 2-pentylcyclopentanone. On agite à 60 °C jusqu'à conversion d'au moins 90% de la 2-pentylcyclopentanone. On laisse le milieu réactionnel revenir à température ambiante. Puis on le verse sur une solution de HCl à 10%. Les phases sont séparées. La phase aqueuse est extraite deux fois au MTBE. Les phases organiques réunies sont lavées avec une solution aqueuse saturée de bicarbonate de sodium puis à l'eau salée. Après séchage sur sulfate de magnésium, filtration sur papier et évaporation du solvant, le produit brut est placé dans du MTBE au réfrigérateur pendant une nuit. Le précipité est filtré sur fritté et rincé au MTBE. Le filtrat est concentré puis le produit brut constitué de deux isomères du 4-(2-pentylcyclopentylidène)-butanenitrile en proportion 52 : 48, est distillé sous pression réduite (T°éb. = 98 °C/66,66 Pascal (0,5 torr).

Puis, on place dans un ballon 1 équivalent de 4-(2-pentylcyclopentylidène)-butanenitrile en proportion 52:48 dans du toluène à environ 10 °C. On ajoute lentement 1,2 équivalents d'une solution de Dibal (hydrure de diisobutylaluminium) à 1.0 M dans le toluène, de manière à garder la température de la solution inférieure à 40 °C. A la fin de l'ajout, le mélange est porté 2 heures à 70 °C, puis laissé à refroidir à température ambiante. Le milieu réactionnel est versé doucement, en agitant, dans un mélange acide acétique/glace. Les phases sont séparées. La phase aqueuse est extraite 3 fois au MTBE. Les phases organiques réunies sont lavées avec une solution saturée de bicarbonate de sodium puis à l'eau salée. Après séchage sur sulfate de magnésium, filtration sur papier et évaporation du solvant, le produit brut, contenant deux isomères en proportion 52:48 de 4-(2-pentylcyclopentylidène)-butanal, est dérésiné par distillation moléculaire, avant d'être distillé plus finement sous pression réduite : sa température d'ébullition est de 75 °C sous 53,32 Pascal (0,4 torr).

Le 4-(2-pentylcyclopentylidène)-butanal ainsi obtenu présente les caractéristiques spectrales suivantes :

### Isomère majoritaire (52 %) :

**MS** [e/m (%)]: 208 (M+, 2), 164 (42), 149 (10), 147 (17), 138 (11), 137 (17), 135 (48), 134 (65), 133 (18), 121 (45), 120 (25), 119 (54), 110 (12), 109 (31), 108 (28), 107 (30), 105 (15), 97 (32), 96 (20), 95 (74), 94 (52), 93 (79), 92 (19), 91 (94), 83 (10), 82 (32), 81 (81), 80 (22), 79 (93), 78 (11), 77 (48), 69 (16), 68 (13), 67 (100), 65 (19), 57 (10), 55 (60), 53 (24), 43 (27), 41 (82), 39 (30).
**¹³C-NMR** (50 MHz, CDCl₃): δ (ppm) 14,12, 22,07, 22,75, 23,98, 30,34, 31,87, 32,05, 33,38, 34,95, 40,29, 44,23, 118,01, 149,15, 202,58.

### Isomère minoritaire (48 %) :

**MS** [e/m (%)]: 208 (M+, 23), 164 (39), 147 (17), 137 (18), 135 (46), 134 (65), 133 (18), 121 (44), 120 (27), 119 (60), 110 (13), 109 (30), 108 (29), 107 (29), 105 (17), 97 (33), 96 (20), 95 (79), 94 (55), 93 (86), 92 (20), 91 (100), 82 (32), 81 (84), 80 (22), 79 (97), 78 (13), 77 (50), 69 (16), 68 (12), 67 (100), 65 (19), 57 (10), 55 (63), 53 (25), 43 (28), 41 (85), 39 (29).
**¹³C-NMR** (50 MHz, CDCl₃) : δ (ppm) 14,12, 22,37, 22,71, 24,09, 29,28, 30,34, 32,16, 32,71, 34,44, 43,90, 44,34, 117,11, 148,79, 202,73.

### 2 isomères superposés :

**¹H-NMR** (200 MHz, CDCl₃) : δ (ppm) 0,89 (t, *J* = 8,0 Hz, 3H), 1,28 (m, 7H), 1,43-1,63 (m, 3H), 1,70-1,91 (m, 2H), 2,18-2,40 (m, 4H), 2,45-2,53 (m, 3H), 5,10-5,17 (m, 1H), 9,77 (2t superposés, *J* = 2,0 Hz, 1H).
**IR** (film, cm⁻¹) : 839w, 1053w, 1379w, 1466m, 1726s, 2714w, 2856m, 2926s, 2953s.

### Exemple 2 : Préparation du 4-(3,3-diméthylcyclohexylidène)-butanal

Le 4-(3,3-diméthylcyclohexylidène)-butanal est préparé selon le protocole décrit en Exemple 1 en utilisant la 3,3-diméthylcyclohexanone à la place de la 2-pentylcyclopentanone. Le produit brut constitué de deux isomères de 4-(3,3-diméthylcyclohexylidène)-butanal, en proportion 74:26, est distillé sous pression réduite : sa température d'ébullition est de 85 °C sous 53,32 Pascal (0,4 torr).

Le 4-(3,3-diméthylcyclohexylidène)-butanal ainsi obtenu présente les caractéristiques spectrales suivantes :

### Isomère majoritaire (74 %) :

**¹H-NMR** (200 MHz, CDCl₃) : δ (ppm) 0,84 (s, 6H), 1,33-1,36 (m, 2H), 1,43-1,56 (m, 2H), 1,82 (s, 2H), 1,96-2,10 (m, 2H), 2,29-2,51 (m, 4H), 5,00 (t, *J* = 7,0 Hz, 1H), 9,76 (t, *J* = 2,0 Hz, 1H).
¹³C-NMR (50 MHz, CDCl₃): δ (ppm) 20,02, 22,88, 27,88, 28,07, 36,34, 39,30, 44,07, 49,99, 120,04, 138,88, 202,37.
**MS** [e/m (%)]: 180 (M+, 1), 165 (12), 162 (26), 147 (38), 137 (11), 136 (32), 121 (35), 119 (12), 109 (39), 107 (19), 105 (15), 97 (18), 96 (10), 95 (25), 93 (40), 91 (27), 82 (13), 81 (56), 80 (13), 79 (36), 77 (25), 70 (10), 69 (100), 68 (20), 67 (37), 65 (12), 55 (40), 53 (22), 43 (14), 41 (72), 39 (28).

### Isomère minoritaire (26 %) :

**¹H-NMR** (200 MHz, CDCl₃) : δ (ppm) 0,88 (s, 6H), 1,33-1,36 (m, 2H), 1,43-1,56 (m, 2H), 1,91 (s, 2H), 1,96-2,10 (m, 2H), 2,29-2,51 (m, 4H), 5,15 (t, *J* = 7,0 Hz, 1H), 9,76 (t, *J* = 2,0 Hz, 1H).
¹³C-NMR (50 MHz, CDCl₃) : δ (ppm) 19,79, 23,81, 28,21, 32,42, 35,57, 39,51, 41,61, 49,99, 120,12, 139,06, 202,37.
**MS** [e/m (%)]: 180 (M+, 1), 162 (24), 147 (35), 136 (29), 121 (33), 119 (12), 109 (36), 107 (15), 105 (15), 97 (18), 95 (22), 93 (38), 91 (24), 82 (10), 81 (48), 80 (11), 79 (31), 77 (21), 70 (10), 69 (100), 68 (18), 67 (31), 65 (10), 55 (38), 53 (18), 43 (12), 41 (65), 39 (23) .
**IR** (film, cm⁻¹): 859w, 975w, 1053w, 1365m, 1456m, 1725s, 2715w, 2841m, 2865m, 2925s, 2948s.

### Exemple 3 : Préparation du 4-(2,4,4-triméthylcyclopentylidène)-butanal

Le 4-(2,4,4-triméthycyclopentylidène)-butanal est préparé selon le protocole décrit en Exemple 1 en utilisant la 2,4,4-triméthylcyclopentanone à la place de la 2-pentylcyclopentanone. Le produit brut constitué de deux isomères du 4-(2,4,4-triméthycyclopentylidène)-butanal en proportions (70:30), est distillé sous pression réduite : sa température d'ébullition est de 70 °C sous 53,32 Pascal (0,4 torr).

Le 4-(2,4,4-triméthycyclopentylidène)-butanal ainsi obtenu présente les caractéristiques spectrales suivantes :

### Isomère majoritaire (70 %) :

**¹H-NMR** (200 MHz, CDC1₃): δ (ppm) 1,02 (s, 6H), 0,91-1,06 (d superposés, 3H), 1,10-1,15 (m, 1H), 1,64-1,86 (m, 2H), 2,02-2,47 (m, 5H), 2,62-2,80 (m, 1H), 5,07-5,12 (m, 1H), 9,76 (t, *J* = 1,6 Hz, 1H).
¹³C-NMR (50 MHz, CDCl₃) : δ (ppm) 21,34, 21,43, 26,83, 28,81, 33,85, 37,69, 44,15, 48,96, 50,00, 118,90, 149,58, 202,49.
**MS** [e/m (%)]: 180 (M+, 1), 162 (18), 147 (41), 137 (13), 136 (68), 123 (12), 122 (11), 121 (100), 119 (13), 111 (16), 109 (34), 107 (19), 105 (15), 97 (11), 95 (37), 93 (33), 91 (28), 81 (37), 79 (33), 77 (26), 69 (15), 68 (12), 67 (32), 65 (11), 55 (34), 53 (20), 43 (12), 41 (47), 39 (25) .

### Isomère minoritaire (30 %) :

**¹H-NNR** (200 MHz, CDCl₃) : δ (ppm) 0,81 (s, 6H), 0,91-1,06 (d superposés, 3H), 1,10-1,15 (m, 1H), 1,64-1,86 (m, 2H), 2,02-2,47 (m, 5H), 2,62-2,80 (m, 1H), 5,07-5,12 (m, 1H), 9,76 (t, *J* = 1,6 Hz, 1H).
**¹³C-NMR** (50 MHz, CDCl₃) : δ (ppm) 19,40, 22,32, 28,06, 29,51, 37,02, 37,12, 43,84, 44,46, 49,73, 117,57, 149,61, 202,62.
**MS** [e/m (%)]:180 (M+, 1), 162 (18), 147 (37), 137 (14), 136 (72), 123 (12), 122 (11), 121 (100), 119(12), 109 (34), 107 (18), 105 (14), 97 (10), 95 (36), 93 (31), 91 (27), 81 (35), 79 (31), 77 (24), 69 (14), 68 (11), 67 (30), 65 (11), 55 (32), 53 (18), 43 (12), 41 (44), 39 (23).
IR (film, cm⁻¹) : 830w, 1365m, 1460m, 1725s, 2716w, 2866m, 2927s, 2951s.

### Exemple 4 : Préparation du 5-(2,4,4-triméthylcyclopentylidène)-pentanal

Dans un ballon, on place un équivalent de 5-bromopentanol et un équivalent de triphénylphosphine dans l'éthanol à reflux. Après 72 heures d'agitation dans ces conditions, et une fois revenu à température ambiante, l'éthanol est évaporé sous vide et le résidu est placé dans du toluène à 4 °C une nuit. Le bromure de (5-hydroxypentyl)triphénylphosphonium formé est filtré sur fritté, rincé au méthyle tert-butyle éther puis séché sous vide.

On place dans un ballon 1 équivalent de bromure de (5-hydroxypentyl)triphénylphosphonium et 1,2 équivalents de *tertio*-butylate de potassium dans du toluène sec. On agite cette suspension à 70 °C pendant 2 heures. Puis, on revient à température ambiante avant d'ajouter la 2,4,4-triméthylcyclopentanone. On agite à 70 °C jusqu'à conversion d'au moins 90 %. On laisse le milieu réactionnel revenir à température ambiante. Puis on le verse sur une solution de HCl à 10 %. Les phases sont séparées. La phase organique est lavée avec une solution aqueuse saturée de bicarbonate de sodium puis à l'eau salée. Après séchage sur sulfate de magnésium, filtration sur papier et évaporation du solvant, le produit brut est placé dans du MTBE au réfrigérateur pendant une nuit. Le précipité est filtré sur fritté et rincé au MTBE. Le filtrat est concentré puis le produit brut constitué de deux isomères du 5-(2,4,4-triméthylcyclopentylidène)-pentan-1-ol en proportion 68:32, est distillé sous pression réduite (T°éb. = 66 °C/26,66 Pascal (0,2 torr)).

Ensuite, on place dans le ballon 2,5 équivalents de PDC (pyridinium dichromate) et 1 équivalent de 5-(2,4,4-triméthylcyclopentylidène)-pentan-l-ol en proportion 68:32 dans du dichlorométhane. On agite vigoureusement la suspension à température ambiante pendant une nuit. Lorsque la conversion est satisfaisante (> 95 %), on filtre le milieu réactionnel sur Célite® puis sur silice. Le filtrat ainsi obtenu est lavé avec une solution aqueuse de HCl à 1 %, puis avec une solution aqueuse saturée de bicarbonate de sodium puis à l'eau. Après séchage sur sulfate de magnésium, filtration sur papier et évaporation du solvant, le produit brut, contenant deux isomères en proportion 68:32 de 5-(2,4,4-triméthylcyclopentylidène)-pentanal, est distillé sous pression réduite : sa température d'ébullition est de 52 °C sous 39,99 Pascal (0,3 torr).

Le 5-(2,4,4-triméthylcyclopentylidène)-pentanal ainsi obtenu présente les caractéristiques spectrales suivantes :

### Isomère majoritaire (68 %) :

**¹H-NMR** (200 MHz, CDCl₃) : δ (ppm) 0,84 (s, 3H), 1,02-1,06 (s superposés, 6H), 1,11-1,17 (m, 1H), 1,64-1,77 (m, 4H), 1,81-2,08 (m, 3H), 2,40-2,45 (m, 2H), 2,66-2,64 (m, 1H), 5,09-5,16 (m, 1H), 9,77 (t, *J* = 1,8 Hz, 1H).
¹³C-NMR (50 MHz, CDCl₃) : δ (ppm) 21,21, 22,41, 26,88, 27,69, 28,82, 33,82, 36,94, 43,37, 49,02, 50,03, 120,13, 149,08, 202,73.
**MS** [e/m (%)]: 194 (M+, 12), 179 (29), 176 (13), 161 (61), 151 (21), 150 (66), 137 (10), 135 (80), 133 (22), 123 (24), 121 (25), 119 (31), 111 (16), 110 (12), 109 (56), 108 (15), 107 (38), 105 (19), 96 (10), 95 (100), 94 (18), 93 (36), 91 (35), 83 (25), 82 (13), 81 (67), 80 (10), 79 (72), 77 (33), 70 (10), 69 (34), 68 (10), 67 (47), 65 (14), 57 (10), 55 (42), 53 (24), 43 (16), 41 (56), 39 (22).

### Isomère minoritaire (32 %) :

**¹H-NMR** (200 MHz, CDCl₃) : δ (ppm) 0,93 (s, 3H), 1,02-1,06 (s superposés, 6H), 1,11-1,17 (m, 1H), 1,64-1,77 (m, 4H), 1,81-2,08 (m, 3H), 2,40-2,45 (m, 2H), 2,66-2,64 (m, 1H), 5,33-5,40 (1H), 9,77 (t, *J* = 1,8 Hz, 1H).
¹³C-NMR (50 MHz, CDCl₃) : δ (ppm) 19,51, 22,15, 28,04, 28,67, 29,51, 37,09, 37,64, 43,28, 44,48, 49,76, 118,70, 149,33, 202,73.
**MS** [e/m (%)]: 194 (M+, 13), 179 (31), 176 (145), 161 (69), 151 (20), 150 (64), 137 (11), 135 (76), 133 (24), 123 (24), 121 (24), 119 (36), 111 (20), 110 (12), 109 (59), 108 (17), 107 (38), 105 (22), 95 (100), 94 (17), 93 (37), 91 (38), 83 (24), 82 (12), 81 (70), 79 (72), 77 (35), 70 (12), 69 (37), 68 (12), 67 (46), 65 (14), 57 (10), 55 (41), 53 (25), 43 (17), 41 (58), 39 (26).
**IR** (film, cm⁻¹): 838w, 1365m, 1459m, 1726s, 2715w, 2866m, 2928s, 2951s.

### Exemple 5 : Préparation du 5-cycloheptylidènepentanal

Dans un ballon, on place un équivalent de pivalate de 5-bromopentyle et un équivalent de triphénylphosphine dans le toluène à 110 °C. Après 72 heures d'agitation dans ces conditions, et une fois revenu à température ambiante, le toluène est évaporé et le bromure de triphényl(5-(pivaloyloxy)pentyl)phosphonium formé est utilisé directement pour l'étape suivante.

On place dans un ballon 1 équivalent de bromure de triphényl(5-(pivaloyloxy)pentyl)phosphonium et 1,2 équivalents de tertio-butylate de potassium dans du THF sec. On agite cette suspension à 60 °C pendant 2 heures. Puis, on ajoute la cycloheptanone. On agite à 60 °C jusqu'à conversion d'au moins 90 %. On laisse le milieu réactionnel revenir à température ambiante, puis on le verse sur une solution de HCl à 10 %. Les phases sont séparées. La phase aqueuse est extraite deux fois au MTBE. Les phases organiques sont lavées avec une solution aqueuse saturée de bicarbonate de sodium puis à l'eau salée. Après séchage sur sulfate de magnésium, filtration sur papier et évaporation du solvant, le produit brut est placé dans du MTBE au réfrigérateur pendant une nuit. Le précipité est filtré sur fritté et rincé au MTBE. Le filtrat est concentré puis le produit brut constitué du pivalate de 5-cycloheptylidènepentyle, est distillé sous pression réduite (Téb. = 104 °C / 93,32 Pascal (0,7 torr).

Le pivalate de 5-cycloheptylidènepentyle est ensuite placé avec 0,1 équivalent d'hydroxyde de potassium au reflux de l'éthanol durant vingt heures. On laisse alors le milieu réactionnel revenir à température ambiante. Puis on le verse sur une solution de HCl à 10 %. Les phases sont séparées. La phase aqueuse est extraite deux fois au MTBE. Les phases organiques sont lavées avec une solution aqueuse saturée de bicarbonate de sodium puis à l'eau salée. Après séchage sur sulfate de magnésium, filtration sur papier et évaporation du solvant, le produit brut constitué du 5-cycloheptylidènepentan-1-ol, est distillé sous pression réduite (T°éb. = 78 °C/79,99 Pascal (0,6 torr).

L'alcool ainsi obtenu est ensuite oxydé (cf. par exemple la dernière étape de l'Exemple 4) pour donner le 5-cycloheptylidènepentanal après purification sous pression réduite : sa température d'ébullition est de 80 °C sous 53,32 Pascal (0,4 torr).

Le 5-cycloheptylidènepentanal ainsi obtenu présente les caractéristiques spectrales suivantes :
**¹H-NMR** (200 MHz, CDCl₃) : δ (ppm) 0,93 (s, 3H), 1,02-1,06 (s superposés, 6H), 1,11-1,17 (m, 1H), 1,64-1,77 (m, 4H), 1,81-2,08 (m, 3H), 2,40-2,45 (m, 2H), 2,66-2,64 (m, 1H), 5,33-5,40 (1H), 9,77 (t, *J* = 1,8 Hz, 1H). ¹³C-NMR (50 MHz, CDCl₃) : δ (ppm) 19,51, 22,15, 28,04, 28,67, 29,51, 37,09, 37,64, 43,28, 44,48, 49,76, 118,70, 149,33, 202,73.
**MS** [e/m (%)]: 180 (M+, 3), 162 (14), 137 (11), 136 (69), 121 (64), 111 (16), 108 (40), 107 (39), 98 (13), 96 (10), 95 (58), 94 (31), 93 (39), 91 (21), 82 (42), 81 (94), 80 (23), 79 (64), 77 (27), 69 (22), 68 (28), 67 (100), 66 (10), 65 (15), 55 (59), 54 (23), 53 (29), 41 (76), 39 (36).
**IR** (film, cm⁻¹) : 1057w, 1442m, 1724s, 2713w, 2850m, 2919s.

### Exemple 6 : Préparation du 5-(4,4-diéthylcyclohexylidène)-pentanal

Le 5-(4,4-diéthylcyclohexylidène)-pentanal est préparé selon le protocole décrit dans l'Exemple 5 en utilisant la 4,4-diéthylcyclohexanone à la place de la cycloheptanone. Le produit brut est distillé sous pression réduite : sa température d'ébullition est de 80 °C sous 53,32 Pascal (0,4 torr).

Le 5-(4,4-diéthylcyclohexylidène)-pentanal ainsi obtenu présente les caractéristiques spectrales suivantes :
**¹H-NMR** (200 MHz, CDCl₃): δ (ppm) 0,93 (s, 3H), 1,04-1,06 (s superposés, 6H), 1,34-1,46 (m, 2H), 1,56-2,05 (m, 7H), 2,16-2,23 (m, 1H), 2,43 (t, *J* = 7,4 Hz, 2H), 2,66 (q, *J* = 7,0 Hz, 1H), 5,10-5,18 (m, 1H), 9,75 (t, *J* = 1,8 Hz, 1H).
¹³C-NMR (50 MHz, CDC1₃): δ (ppm) 19,49, 21,59, 26,81, 28,79, 28,97, 29,14, 36,85, 36,97, 44,39, 48,94, 49,78, 119,67, 148,15, 202,34.
**MS** [e/m (%)]: 208 (M+, 19), 193 (59), 176 (13), 175 (84), 165 (22), 147 (15), 135 (12), 133 (11), 125 (10), 124 (10), 123 (44), 122 (13), 121 (21), 119 (20), 111 (10), 110 (16), 109 (99), 108 (14), 107 (38), 105 (15), 95 (100), 93 (42), 91 (33), 84 (15), 83 (16), 82 (16), 81 (69), 80 (13), 79 (38), 77 (32), 69 (33), 67 (46), 66 (11), 55 (28), 53 (18), 52 (10), 43 (15), 41 (41), 39 (14).
**IR** (film, cm⁻¹): 844w, 1365m, 1459m, 1727s, 2714w, 2865m, 2927s, 2950s.

### Exemple 7 : Préparation du 6-(2,4,4-triméthylcyclopentylidène)-hexanal

Dans un ballon, on place un équivalent d'acétate de bromohexyle et un équivalent de triphénylphosphine dans l'éthanol à reflux. Après 72 heures d'agitation dans ces conditions, et une fois revenu à température ambiante, l'éthanol et l'acétate d'éthyle formé sont évaporés sous vide et le résidu est placé dans du toluène à 4°C une nuit. Le bromure de (6-hydroxypentyl)triphénylphosphonium formé est filtré sur fritté, rincé au méthyle tert-butyle éther (MTBE) puis séché sous vide.

On place dans un ballon 1 équivalent de bromure de (6-hydroxyhexyl)triphénylphosphonium et 1,2 équivalents de tertio-butylate de potassium dans du toluène sec. On agite cette suspension à 70 °C pendant 2 heures, puis on ajoute la 2,4,4-triméthylcyclopentanone. On agite à 70 °C jusqu'à conversion d'au moins 90 %. On laisse le milieu réactionnel revenir à température ambiante. Puis on le verse sur une solution de HCl à 10 %. Les phases sont séparées. La phase organique est lavée avec une solution aqueuse saturée de bicarbonate de sodium puis à l'eau salée. Après séchage sur sulfate de magnésium, filtration sur papier et évaporation du solvant, le produit brut est placé dans du MTBE au réfrigérateur pendant une nuit. Le précipité est filtré sur fritté et rincé au MTBE. Le filtrat est concentré puis le produit brut constitué de deux isomères du 6-(2,4,4-triméthylcyclopentylidène)-hexan-1-ol en proportion 70:30, est distillé sous pression réduite (T°éb. = 89 °C/13,33 Pascal (0,1 torr).

Ensuite, on place dans un ballon 2,5 équivalents de PDC (pyridinium dichromate) et 1 équivalent de 6-(2,4,4-triméthylcyclopentylidène)-hexan-l-ol en proportion 70:30 dans du dichlorométhane. On agite vigoureusement la suspension à température ambiante pendant une nuit. Lorsque la conversion est satisfaisante (> 95 %), on filtre le milieu réactionnel sur Célite® puis sur silice. Le filtrat ainsi obtenu est lavé avec une solution aqueuse de HCl à 1 %, puis avec une solution aqueuse saturée de bicarbonate de sodium puis à l'eau. Après séchage sur sulfate de magnésium, filtration sur papier et évaporation du solvant, le produit brut contenant deux isomères en proportion 70:30 de 6-(2,4,4-triméthylcyclopentylidène)-hexanal est distillé sous pression réduite : sa température d'ébullition est de 59 °C sous 26,66 Pascal (0,2 torr).

Le 6-(2,4,4-triméthylcyclopentylidène)-hexanal ainsi obtenu présente les caractéristiques spectrales suivantes :

### Isomère majoritaire (70 %) :

**¹H-NMR** (200 MHz, CDC1₃): δ (ppm) 0,83 (s, 3H), 1,04-1,06 (s superposés, 6H), 1,34-1,46 (m, 2H), 1,56-2,05 (m, 7H), 2,16-2,23 (m, 1H), 2,42 (t, *J* = 7,2 Hz, 2H), 2,66 (q, *J* = 7,0 Hz, 1H), 5,10-5,18 (m, 1H), 9,75 (t, *J* = 1,8 Hz, 1H).
¹³C-NMR (50 MHz, CDCl₃) : δ (ppm) 21,45, 21,67, 26,81, 28,03, 28,79, 29,47, 33,74, 37,58, 43,70, 48,94, 50,04, 120,81, 147,99, 202,29.
**MS** [e/m (%)]: 208 (M+, 8), 194 (10), 193 (50), 190 (22), 175 (63), 165 (18), 147 (14), 137 (13), 133 (13), 124 (11), 123 (55), 122 (12), 121 (25), 119 (18), 110 (18), 109 (74), 108 (16), 107 (25), 105 (20), 95 (100), 93 (36), 91 (31), 83 (12), 81 (52), 79 (41), 77 (25), 69 (32), 68 (10), 67 (33), 65 (13), 55 (30), 53 (19), 43 (16), 41 (52), 39 (16).

### Isomère minoritaire (30 %) :

**¹H-NMR** (200 MHz, CDCl₃) : δ (ppm) 0,93 (s, 3H), 1,04-1,06 (s superposés, 6H), 1,34-1,46 (m, 2H), 1,56-2,05 (m, 7H), 2,16-2,23 (m, 1H), 2,43 (t, *J* = 7,4 Hz, 2H), 2,66 (q, *J* = 7,0 Hz, 1H), 5,10-5,18 (m, 1H), 9,75 (t, *J* = 1,8 Hz, 1H).
¹³C-NMR (50 MHz, CDCl₃) : δ (ppm) 19,49, 21,59, 26,81, 28,79, 28,97, 29,14, 36,85, 36,97, 44,39, 48,94, 49,78, 119,67, 148,15, 202,34.
**MS** [e/m (%)]: 208 (M+, 19), 193 (59), 176 (13), 175 (84), 165 (22), 147 (15), 135 (12), 133 (11), 125 (10), 124 (10), 123 (44), 122 (13), 121 (21), 119 (20), 111 (10), 110 (16), 109 (99), 108 (14), 107 (38), 105 (15), 95 (100), 93 (42), 91 (33), 84 (15), 83 (16), 82 (16), 81 (69), 80 (13), 79 (38), 77 (32), 69 (33), 67 (46), 66 (11), 55 (28), 53 (18), 52 (10), 43 (15), 41 (41), 39 (14).
**IR** (film, cm⁻¹) : 844w, 1365m, 1459m, 1727s, 2714w, 2865m, 2927s, 2950s.

### Exemple 8 : Préparation du 6-(2-méthylcyclohexylidène)-hexanal

Le 6-(2-méthylcyclohexylidène)-hexanal est préparé selon le protocole décrit en Exemple 7 en utilisant la 2-méthylcyclohexanone à la place de la 2,4,4-triméthylcyclopentanone. Le produit brut constitué de deux isomères en proportion 70:30, est distillé sous pression réduite : sa température d'ébullition est de 71 °C sous 39,99 Pascal (0,3 torr).

Le 6-(2-méthylcyclohexylidène)-hexanal ainsi obtenu présente les caractéristiques spectrales suivantes :

### Isomère majoritaire (70 %) :

**¹H-NMR** (200 MHz, CDCl₃): δ (ppm) 0,99-1,06 (d superposés, 3H), 1,33-1,44 (m, 5H), 1,48-1,71 (m, 7H), 1,98-2, 09 (m, 3H), 2,44 (td, *J* = 6,0 Hz, *J* = 1,8 Hz, 2H), 4,96-5,06 (m, 1H), 9,76 (t, *J* = 1,8 Hz, 1H).
¹³C-NMR (50 MHz, CDCl₃): δ (ppm) 18,63, 21,63, 25,45, 28,10, 28,15, 29,70, 36,74, 38,37, 43,77, 117,90, 144,07, 202,73.
**MS** [e/m (%)]: 194 (M+, 9), 176 (15), 161 (20), 147 (15), 110 (10), 109 (50), 108 (22), 107 (11), 97 (12), 96 (67), 95 (81), 94 (14), 93 (25), 91 (19), 82 (15), 81 (100), 80 (13), 79 (25), 77 (18), 69 (14), 68 (15), 67 (75), 65 (11), 55 (44), 53 (16), 41 (39), 39 (17).

### Isomère minoritaire (30 %) :

**¹H-NMR** (200 MHz, CDCl₃) : δ (ppm) 0,99-1,06 (d superposés, 3H), 1,33-1,44 (m, 5H), 1,48-1,71 (m, 7H), 1,98-2,09 (m, 3H), 2,44 (td, *J* = 6,0 Hz, *J* = 1,8 Hz, 2H), 4,96-5,06 (m, 1H), 9,76 (t, *J* = 1,8 Hz, 1H).
¹³C-NMR (50 MHz, CDCl₃): δ (ppm) 18,12, 20,93, 26,38, 26,69, 28,56, 29,62, 30,13, 32,44, 33,19, 120,55, 143,59, 202,73.
**MS** [e/m (%)]: 194 (M+, 5), 176 (19), 161 (21), 147 (17), 133 (12), 110 (10), 109 (47), 108 (22), 107 (11), 97 (13), 96 (39), 95 (76), 94 (15), 93 (27), 91 (19), 82 (14), 81 (100), 80 (12), 79 (41), 77 (18), 69 (14), 68 (15), 67 (75), 55 (45), 53 (16), 41 (41), 39 (15).
**IR** (film, cm⁻¹): 896w, 1368w, 1456m, 1725s, 2714w, 2852m, 2923s.

### Exemple 9 : Préparation du 6-(4-méthylcyclohexylidène)-hexanal

Le 6-(4-méthylcyclohexylidène)-hexanal est préparé selon le protocole décrit en Exemple 7 en utilisant la 4-méthylcyclohexanone à la place de la 2,4,4-triméthylcyclopentanone. Le produit brut contenant le 6-(4-méthylcyclohexylidène)-hexanal est distillé sous pression réduite : sa température d'ébullition est de 69 °C sous 46,66 Pascal (0,35 torr).

Le 6-(4-méthylcyclohexylidène)-hexanal ainsi obtenu présente les caractéristiques spectrales suivantes :
**¹H-NMR** (200 MHz, CDCl₃) : δ (ppm) 0,89 (d, *J* = 6,6 Hz, 3H), 0,98-1,02 (m, 1H), 1,35-1,40 (m, 3H), 1,58-1,77 (m, 6H), 1,99-2,11 (m, 4H), 2,42-2,57 (m, 3H), 5,05 (t, *J* = 7,2 Hz, 2H), 9,76 (s, 1H).
¹³C-NMR (50 MHz, CDC1₃): δ (ppm) 21,59, 22,05, 26,78, 27,88, 29,58, 32,84, 35,97, 36,41, 36,81, 43,77, 120,67, 139,63, 202,71.
**MS** [e/m (%)]: 194 (M+, 5), 176 (35), 161 (13), 147 (19), 135 (10), 123 (13), 109 (11), 108 (13), 107 (14), 96 (10), 95 (65), 94 (22), 93 (30), 91 (18), 82 (10), 81 (100), 80 (12), 79 (40), 77 (22), 69 (13), 68 (21), 67 (55), 65 (12), 55 (36), 53 (17), 41 (40), 39 (18).
**IR** (film, cm⁻¹) : 847w, 1081w, 1373w, 1456m, 1725s, 2714w, 2846m, 2912s, 2948s.

### Exemple 10 : Préparation du 6-(4-tert-butylcyclohexylidène)-hexanal

Le 6-(4-*tert*-butylcyclohexylidène)-hexanal est préparé selon le protocole décrit en Exemple 7 en utilisant la 4-*tert*-butylcyclohexanone à la place de la 2,4,4-triméthylcyclopentanone. Le produit brut contenant le 6-(4-*tert*-butylcyclohexylidène)-hexanal est distillé sous pression réduite : sa température d'ébullition est de 100 °C sous 39,99 Pascal (0,3 torr).

Le 6-(4-*tert*-butylcyclohexylidène)-hexanal ainsi obtenu présente les caractéristiques spectrales suivantes :
**¹H-NMR** (200 MHz, CDCl₃) : δ (ppm) 1,03 (s, 9H), 1,10-1,16 (m, 2H), 1,32-1,44 (m, 3H), 1,56-1,68 (m, 3H), 1,80-1,86 (m, 2H), 1,99-2,03 (m, 3H), 2,16-2,24 (m, 1H), 2,42 (td, *J* = 7,2 Hz, *J* = 1,8 Hz, 2H), 2,61 (dq, *J* = 13,6 Hz, *J* = 2,4 Hz,1H), 5,04 (t, *J* = 7,2 Hz, 1H), 9,76 (t, *J* = 1,8 Hz, 1H).
¹³C-NMR (50 MHz, CDCl₃) : δ (ppm) 21,63, 26,77, 27,59, 28,42, 28,47, 29,23, 29,59, 32,41, 36,96, 43,78, 48,46, 120,19, 140,03, 202,78.
**MS** [e/m (%)]: 236 (M+, 8), 218 (18), 179 (11), 162 (20), 147 (20), 135 (11), 133 (12), 123 (13), 121 (10), 119 (17), 109 (25), 107 (10), 105 (15), 97 (12), 96 (13), 95 (44), 94 (15), 93 (30), 92 (10), 91 (29), 83 (14), 82 (12), 81 (48), 80 (20), 79 (65), 77 (30), 69 (17), 67 (46), 65 (10), 57 (100), 55 (31), 53 (12), 43 (19), 41 (60), 39 (12).
**IR** (film, cm⁻¹): 848w, 1365m, 1443m, 1726s, 2714w, 2861m, 2940s.

### Exemple 11 : Préparation du 6-(4-tert-amylcyclohexylidène)-hexanal

Le 6-(4-*tert*-amylcyclohexylidène)-hexanal est préparé selon le protocole décrit en Exemple 7 en utilisant la 4-*tert*-amylcyclohexanone à la place de la 2,4,4-triméthylcyclopentanone. Le produit brut contenant le 6-(4-*tert*-amylcyclohexylidène)-hexanal est purifié sur colonne de silice par un éluant composé de 5 % d'hexane et 95 % d'acétate d'éthyle.

Le 6-(4-*tert*-amylcyclohexylidène)-hexanal ainsi obtenu présente les caractéristiques spectrales suivantes :
**¹H-NMR** (200 MHz, CDCl₃): δ (ppm) 0,78 (s, 6H), 0,80 (t, *J* = 7,0 Hz, 3H), 1,00 (qd, J = 12,0 Hz, J = 4,0 Hz, 2H), 2H), 1,20-1,40 (m, 6H), 1,56-1,68 (m, 3H), 1,71-1,80 (m, 3H), 1,99-2,05 (m, 3H), 2,16-2,65 (m, 2H), 2,42 (td, *J* = 8,0 Hz, *J* = 2,0 Hz, 2H), 5,03 (t, *J* = 7,0 Hz, 1H), 9,76 (t, *J* = 2,0 Hz, 1H).
¹³C-NMR (50 MHz, CDCl₃): δ (ppm) 8,14, 21,67, 24,36, 26,81, 28,05, 28,59, 28,85, 29,64, 29,79, 32,74, 34,73, 37,09, 43,84, 45,66, 120,19, 140,18, 202,86.
**MS** [e/m (%)]: 250 (M+, 1), 162 (15), 161 (23), 147 (13), 119 (11), 109 (15), 105 (10), 95 (28), 94 (13), 93 (21), 91 (23), 81 (43), 80 (15), 79 (40), 77 (15), 71 (100), 70 (26), 69 (14), 67 (40), 55 (33), 53 (10), 43 (82), 41 (51), 39 (12).

### Exemple 12 : Préparation du 6-cyclooctylidènehexanal

Le 6-cyclooctylidènehexanal est préparé selon le protocole décrit en Exemple 7 en utilisant la cyclooctanone à la place de la 2,4,4-triméthylcyclopentanone. Le produit brut contenant le cyclooctylidènehexanal est distillé sous pression réduite : sa température d'ébullition est de 94 °C sous 26,66 Pascal (0,2 torr).

Le 6-cyclooctylidènehexanal ainsi obtenu présente les caractéristiques spectrales suivantes :
**¹H-NMR** (200 MHz, CDCl₃): δ (ppm) 1,25-1,69 (m, 14H), 2,00-2,17 (m, 6H), 2,43 (td, *J* = 10,0 Hz, *J* = 2,0 Hz, 2H), 5,09-5,58 (m, 1H), 9,77 (t, *J* = 2,0 Hz, 1H).
¹³C-NMR (50 MHz, CDCl₃): δ (ppm) 19,74, 24,14, 26,30, 26,34, 26,57, 28,92, 29,21, 29,77, 32,04, 47,83, 65,58, 125,14, 142,69, 211,33.
**MS** [e/m (%)]: 208 (M+, 9), 190 (13), 162 (41), 161 (10), 147 (13), 135 (12), 133 (12), 122 (15), 121 (15), 110 (11), 109 (51), 108 (15), 107 (21), 97 (20), 96 (40), 95 (81), 94 (25), 93 (28), 91 (25), 84 (11), 83 (20), 82 (36), 81 (100), 80 (26), 79 (57), 77 (23), 70 (10), 69 (28), 68 (23), 67 (94), 65 (13), 55 (60), 54 (16), 53 (24), 43 (13), 41 (70), 39 (26).
**IR** (film, cm⁻¹): 971w, 1447m, 1468m, 1726s, 2712w, 2852m, 2920s.

### Exemple 13 : Préparation du 6-(3,3-diméthylcyclohexylidène)hexanal

Le 6-(3,3-diméthylcyclohexylidène)hexanal est préparé selon le protocole décrit en Exemple 7 en utilisant la 3,3-diméthylcyclohexanone à la place de la 2,4,4-triméthylcyclopentanone. Le produit brut, constitué de deux isomères en proportion 60:40, est distillé sous pression réduite : sa température d'ébullition est de 75 °C sous 61,32 Pascal (0,46 torr).

Le 6-(3,3-diméthylcyclohexylidène)hexanal ainsi obtenu présente les caractéristiques spectrales suivantes :

### Isomère majoritaire (60 %) :

¹H-NMR (200 MHz, CDCl₃): δ (ppm) 0,82 (s, 6H), 1,25-1,5 (m, 6H), 1,5-1,75 (m, 3H), 1,79 (s, 2H), 1,9-2,1 (m, 3H), 2,40 (dt, *J* = 7,36, 1,83 Hz, 2H), 4,97 (t, *J* = 7,32 Hz, 1H), 9,73 (t, *J* = 1,86 Hz, 1H).
¹³C-NMR (50 MHz, CDCl₃): δ (ppm) 21,57; 23,19; 26,83; 28,10; 28,32; 29,61; 32,56; 39,62; 43,73; 50,36; 122,12; 137,81; 202,7.

### Isomère minoritaire (40 %) :

¹H-NMR (200 MHz, CDCl₃): δ (ppm) 0,85 (s, 6H), 1,25-1,5 (m, 6H), 1,5-1,75 (m, 3H), 1,86 (s, 2H), 1,9-2,1 (m, 3H), 2,40 (dt, *J* = 7,36, 1,83 Hz, 2H), 5,12 (tt, *J* = 7,24 Hz, 1,1 Hz, 1H), 9,73 (t, *J* = 1,86 Hz, 1H).
¹³C-NMR (50 MHz, CDCl₃): δ (ppm) 21,72; 24,11; 26,76; 28,45; 29,61; 32,69; 36,66; 39,84; 41,87; 43,76; 122,23; 137,92; 202,7.

### Exemple 14 : Composition parfumante B incorporant le 6-(2,4,4-triméthylcyclopentylidène)hexanal obtenu selon l'Exemple 7

Le test d'une évaluation olfactive comparative étendue à l'étude de l'impact des composés donnés dans diverses formules est effectué comme suit. Une même formule ou accord est réalisée d'une part sans aucune matière première faisant partie des composés de formule (I) revendiqués, d'autre part avec un des composés de formule (I), à un dosage adapté à sa puissance olfactive. Les formules ou accords ainsi préparés font à leur tour l'objet d'une évaluation comparative à l'aveugle.

Les deux accords suivants ont été préparés : un accord tamarine A, puis le même accord tamarine A comprenant en outre le 6-(2,4,4-triméthylcyclopentylidène)-hexanal pour donner l'accord B. Leurs formulations sont décrites ci-dessous et sont utilisées comme bases parfumantes : ils sont incorporés à 1 % en poids dans un gel douche prêt à l'emploi.

| **Ingrédients** | **Accord A** | **Accord B** |
|---|---|---|
| ALDEHYDE C08 | 0,05 | 0,05 |
| LINALOL | 7,40 | 7,40 |
| LIMONENE | 28,00 | 28,00 |
| OXANE ou 2-méthyl-4-propyl-1,3-oxathiane (Firmenich, Suisse) | 0,70 | 0,70 |
| PARACYMENE | 10,00 | 10,00 |
| THYMOL | 0,90 | 0,90 |
| ALDEHYDE C12 LAURIQUE | 0,15 | 0,15 |
| 1,3,5-UNDECATRIENE | 0,20 | 0,20 |
| METHYLE ANTHRANILATE DE METHYLE | 10,00 | 10,00 |
| DIPROPYLENE GLYCOL | 42,60 | 41,70 |
| **6-(2,4,4-triméthylcyclopentylidène)-hexanal** | **0,00** | **0,90** |

L'évaluation comparative des accords A et B à 1 % en poids en base gel douche montre que l'ajout du 6-(2,4,4-triméthylcyclopentylidène)-hexanal à hauteur de 0,9 % dans l'accord B apporte un effet notable et très intéressant : il accentue la facette fruitée mandarine, surtout en cœur par rapport à l'accord A.

### Exemple 15 : Composition parfumante E incluant le 6-(2,4,4-triméthylcyclopentylidène)-hexanal obtenu selon l'Exemple 7

L'évaluation olfactive comparative étendue à l'étude de l'impact des composés donnés en formules est réalisée selon le test décrit dans l'Exemple 7. Dans cet exemple, une troisième formule a été préparée, intégrant une des matières premières comparable olfactivement aux composés de formule (I) : la Calone® (ou 7-méthyl-3,4-dihydro-2H-1,5-benzodioxepin-3-one), en même dosage.

Des eaux de toilette féminines ont été préparées en incorporant 10 % en poids des trois formules parfumantes décrites ci-dessous dont une contient le 6-(2,4,4-triméthylcyclopentylidène)-hexanal et une contient de la Calone® :

| **Ingrédients** | **Accord C** | **Accord D** | **Accord E** |
|---|---|---|---|
| GAMMA NONALACTONE | 0,05 | 0,05 | 0,05 |
| ESSENCE DE CITRON | 2,00 | 2,00 | 2,00 |
| ESSENCE DE BERGAMOTE | 2,00 | 2,00 | 2,00 |
| COUMARINE | 0,30 | 0,30 | 0,30 |
| MUSC T® ou éthylène brassylate (Takasago, Japon) | 5,00 | 5,00 | 5,00 |
| SALICYLATE DE BENZYLE | 18,00 | 18,00 | 18,00 |
| SALICYLATE DE CIS-3-HEXENYLE | 3,00 | 3,00 | 3,00 |
| DIHYDROJASMONATE DE METHYLE | 20,00 | 20,00 | 20,00 |
| VANILLINE | 0,50 | 0,50 | 0,50 |
| GALAXOLIDE® ou 1,3,4,6,7,8-hexahydro-4,6,6,7,8,8-hexaméthylcyclopenta-(g)-2-benzopyranne (IFF, Etats-Unis) | 20,00 | 20,00 | 20,00 |
| ESSENCE D'YLANG | 1,50 | 1,50 | 1,50 |
| ANTHRANILATE DE METHYLE | 0,10 | 0,10 | 0,10 |
| DIPROPYLENE GLYCOL | 27,55 | 25,55 | 25,55 |
| **CALONE®** ou 7-méthyl-3,4-dihydro-2H-1,5-benzodioxepin-3-one (Pfizer and Co., Etats-Unis). | **0,00** | **2,00** | **0,00** |
| **6-(2,4,4-triméthylcyclopentylidène)-hexanal** | **0,00** | **0,00** | **2,00** |

Evaluation comparative des accords C, D et E à 10 % en poids en base alcoolique : l'ajout de 2 % de Calone® dans l'accord D apporte une facette marine. L'ajout de 2 % du 6-(2,4,4-triméthylcyclopentylidène)-hexanal dans l'accord E apporte un effet très intéressant en donnant de la puissance au parfum et particulièrement aux notes musquées, solaires et en apportant plus de rondeur et de poudré par rapport à l'accord C.

### Exemple 16 : Composition parfumante G incorporant le 6-(2,4,4-triméthylcyclopentylidène)-hexanal obtenu selon l'Exemple 7

L'évaluation olfactive comparative étendue à l'étude de l'impact des composés donnés en formules est réalisée selon le test décrit dans l'Exemple 7.

Un accord muguet-poire F, puis le même accord comprenant le 6-(2,4,4-triméthylcyclopentylidène)-hexanal pour donner l'accord G dont les formulations sont décrites ci-dessous, sont utilisés comme bases parfumantes : ils sont préparés puis incorporés à 1 % en poids dans un assouplissant prêt à l'emploi :

| **Ingrédients** | **Accord F** | **Accord G** |
|---|---|---|
| ACETATE D'HEXYLE | 1,10 | 1,10 |
| DIHYDROMYRCENOL® ou 2,6-diméthyl-7-octèn-2-ol (IFF, Etats-Unis) | 0,20 | 0,20 |
| ALCOOL PHENYLETHYLIQUE | 22,20 | 22,20 |
| LINALOL | 6,70 | 6,70 |
| ACETATE DE BENZYLE | 2,20 | 2,20 |
| ACETATE DE STYRALLYLE | 0,20 | 0,20 |
| FRESKOMENTHONE | 0,30 | 0,30 |
| ACETATE DE DIMETHYLBENZYLE CARBINOL | 0,10 | 0,10 |
| ALDEHYDE CYCLAMEN | 3,30 | 3,30 |
| LILIAL® ou para-tert-butyl-alpha-méthylhydrocinnamaldéhyde (Givaudan, Suisse) | 11,00 | 11,00 |
| ALDEHYDE C14 | 0,70 | 0,70 |
| DIHYDROJASMONATE DE METHYLE | 4,50 | 4,50 |
| CITRONELLOL | 13,50 | 13,50 |
| TERPINEOL | 2,20 | 2,20 |
| HELIOTROPINE | 0,80 | 0,80 |
| GALAXOLIDE® ou 1,3,4,6,7,8-hexahydro-4, 6,6,7,8,8-hexaméthylcyclopenta-(g)-2-benzopyranne (IFF, Etats-Unis) | 5,50 | 5,50 |
| PHENOXANOL® ou 3-méthyl-5-phénylpentan-1-ol (IFF, Etats-Unis) | 1,70 | 1,70 |
| ALDEHYDE CINNAMIQUE | 0,20 | 0,20 |
| ALDEHYDE HEXYLE CINNAMIQUE | 3,30 | 3,30 |
| TRIPLAL® ou 2,4-diméthyl-3-cyclohexèn-1-carbaldéhyde (IFF, Etats-Unis) | 0,60 | 0,60 |
| ANTHRANILATE DE METHYLE | 0,10 | 0,10 |
| DIPROPYLENE GLYCOL | 19,60 | 19,50 |
| **6-(2,4,4-triméthylcyclopentylidène)-hexanal** | **0,00** | **0,10** |

L'évaluation comparative des accords F et G à 1 % en poids en base assouplissant, montre que l'ajout de seulement 0,1 % de 6-(2,4,4-triméthylcyclopentylidène)-hexanal en base assouplissant dans l'accord G apporte un effet très notable en accentuant la facette verte, végétale, aqueuse par rapport à l'accord F.

## Revendications

1. Composé de formule générale (I) suivante : 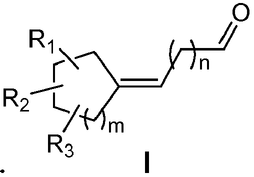 dans laquelle :
- R1, R2 et R3 représentent chacun indépendamment un atome d'hydrogène ou un groupement alkyle en C1-C5 saturé ou insaturé, ramifié ou non ramifié ;
- m est un nombre entier compris entre 1 et 4 ;
- n est un nombre entier compris entre 2 et 4 ;
**caractérisé en ce que** le cycle est saturé et comprend de 5 à 8 carbones, que le nombre total de carbones du cycle et des radicaux R1, R2 et R3 est compris entre 7 et 11
et étant entendu que ledit composé de formule (I) n'est pas :
- le 6-cycloheptylidènehexanal
- le 4-(4-méthylcyclohexylidène)-butanal
- le 4-(4-tert-butylcyclohexylidène)-butanal
- le 4-(3,3,5-triméthylcyclohexylidène)-butanal
- le 4-(3,3-diméthylcyclopentylidène)-butanal
- le 4-(3-éthyl-3-méthylcyclopentylidène)-butanal.

2. Composé selon la revendication 1, **caractérisé en ce que** m est égal à 1.

3. Composé selon la revendication 1 ou 2, **caractérisé en ce que** n est égal à 4.

4. Composé selon la revendication 1 ou 2 **caractérisé en ce que** n est égal à 2.

5. Composé selon la revendication 1, **caractérisé en ce qu'**il est choisi parmi le 5-(2,4,4-triméthylcyclopentylidène)-pentanal, le 6-(2,4,4-triméthylcyclopentylidène)-hexanal, le 6-(2-méthylcyclohexylidène)-hexanal, le 6-(4-méthylcyclohexylidène)-hexanal, le 6-(4-tert-butylcyclohexylidène)-hexanal, le 6-(4-tert-amylcyclohexylidène)-hexanal, le 6-cyclooctylidènehexanal, le 6-(3,3-diméthylcyclohexylidène)hexanal, le 4-(2,4,4-triméthyl cyclopentylidène)butanal, le 4-(2-pentylcyclopentylidène)-butanal, le 4-(3,3-diméthyl cyclohexylidène)-butanal, le 5-(4,4-diéthylcyclohexylidène)-pentanal et le 5-cycloheptylidènepentanal.

6. Composition **caractérisée en ce qu'**elle comprend au moins un composé de formule générale (I) tel que défini dans la revendication 1, sous forme d'isomère ou d'un mélange d'isomères, d'un énantiomère ou d'un mélange d'énantiomères, ou d'un mélange racémique, ou d'un diastéréoisomère ou d'un mélange de diastéréisomères.

7. Composition selon la revendication précédente **caractérisée en ce qu'**elle comprend en outre au moins une autre substance odorante.

8. Procédé de préparation d'un composé de formule (I) tel que décrit dans la revendication 1 dans lequel une cycloalcanone et un ylure de phosphore sont soumis à une réaction de Wittig, suivie d'une étape de réduction et/ou d'oxydation selon l'ylure utilisé précédemment.

9. Procédé selon la revendication 8 **caractérisé en ce qu'**il comprend les étapes de : 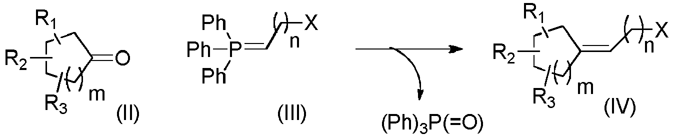
i) addition d'un ylure de phosphore de formule (III) sur une cycloalcanone de formule (II), selon une réaction de Wittig
R1, R2 et R3 représentant chacun indépendamment un atome d'hydrogène ou groupement alkyle en C1-C5 saturé ou insaturé, ramifié ou non ramifié ;
m étant un nombre entier compris entre 1 et 4 ;
n étant un nombre entier compris entre 2 et 4 ;
le cycle étant saturé, comprenant de 5 à 8 carbones, le nombre total de carbones du cycle et des radicaux R1, R2 et R3 étant compris entre 7 et 11 ;
et X représentant une fonction nitrile, ester carboxylique ou alcool ; et
ii) conversion de la fonction X du composé (IV) obtenu, en aldéhyde par réduction et/ou oxydation.

10. Utilisation d'au moins un composé de formule (I) tel que défini dans l'une des revendications 1 à 5 en tant qu'agent ou composé fragrant.

11. Utilisation d'au moins un composé de formule (I) tel que défini dans l'une des revendications 1 à 5 en tant qu'agent masquant d'une odeur ou neutralisant d'une odeur.

12. Utilisation, selon l'une des revendications 10 ou 11, d'au moins un composé de formule (I) seul ou en combinaison avec au moins un autre ingrédient aromatisant ou parfumant, et/ou au moins un solvant, et/ou au moins un adjuvant.

13. Utilisation selon l'une des revendications 10 à 12 pour conférer, modifier ou renforcer les propriétés organoleptiques d'une substance, d'une composition ou d'un article.

14. Utilisation selon l'une des revendications 10 à 13 pour la fabrication de compositions telles que des compositions de parfumerie, des compositions topiques, en particulier cosmétiques, ou encore des produits d'entretien.

## Patentansprüche

1. Eine Verbindung der folgenden allgemeinen Formel (I): 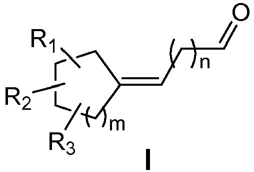 wobei:
- R1, R2 und R3 jeweils unabhängig ein Wasserstoffatom oder eine gesättigte oder ungesättigte, verzweigte oder unverzweigte C1-C5-Alkylgruppe darstellen;
- m eine ganze Zahl zwischen 1 und 4 ist;
- n eine ganze Zahl zwischen 2 und 4 ist;
**dadurch gekennzeichnet, dass** der Ring gesättigt ist und 5 bis 8 Kohlenstoffe umfasst, dass die Gesamtzahl der Kohlenstoffe des Rings und der Reste R1, R2 und R3 zwischen 7 und 11 ist,
und wobei es sich versteht, dass die Verbindung der Formel (I) nicht Folgendes ist:
- 6-Cycloheptylidenhexanal
- 4-(4-Methylcyclohexyliden)-butanal
- 4-(4-tert-Butylcyclohexyliden)-butanal
- 4-(3,3,5-Trimethylcyclohexyliden)-butanal
- 4-(3,3-Dimethylcyclopentyliden)-butanal
- 4-(3-Ethyl-3-methylcyclopentyliden)-butanal.

2. Verbindung gemäß Anspruch 1, **dadurch gekennzeichnet, dass** m gleich 1 ist.

3. Verbindung gemäß Anspruch 1 oder 2, **dadurch gekennzeichnet, dass** n gleich 4 ist.

4. Verbindung gemäß Anspruch 1 oder 2, **dadurch gekennzeichnet, dass** n gleich 2 ist.

5. Verbindung gemäß Anspruch 1, **dadurch gekennzeichnet, dass** sie ausgewählt ist aus Folgendem: 5-(2,4,4-Trimethylcyclopentyliden)-pentanal, 6-(2,4,4-Trimethylcyclopentyliden)-hexanal, 6-(2-Methylcyclohexyliden)-hexanal, 6-(4-Methylcyclohexyliden)-hexanal, 6-(4-tert-Butylcyclohexyliden)-hexanal, 6-(4-tert-Amylcyclohexyliden)-hexanal, 6-Cyclooctylidenhexanal, 6-(3,3-Dimethylcyclohexyliden)-hexanal, 4-(2,4,4-Trimethylcyclopentyliden)butanal, 4-(2-Pentylcyclopentyliden)-butanal, 4-(3,3-Dimethylcyclohexyliden)-butanal, 5-(4,4-Diethylcyclohexyliden)-pentanal und 5-Cycloheptylidenpentanal.

6. Eine Zusammensetzung, **dadurch gekennzeichnet, dass** sie mindestens eine Verbindung der allgemeinen Formel (I), wie in Anspruch 1 definiert, umfasst, in Form eines Isomers oder einer Mischung aus Isomeren, eines Enantiomers oder einer Mischung aus Enantiomeren oder einer racemischen Mischung oder eines Diastereomers oder einer Mischung aus Diastereomeren.

7. Zusammensetzung gemäß dem vorhergehenden Anspruch, **dadurch gekennzeichnet, dass** sie ferner mindestens eine weitere Duftsubstanz umfasst.

8. Ein Verfahren zur Herstellung einer Verbindung der Formel (I), wie in Anspruch 1 beschrieben, wobei ein Cycloalkanon und ein Phosphorylid einer Wittig-Reaktion unterzogen werden, gefolgt von einem Reduktions- und/oder Oxidationsschritt gemäß dem zuvor verwendeten Ylid.

9. Verfahren gemäß Anspruch 8, **dadurch gekennzeichnet, dass** es die folgenden Schritte umfasst: 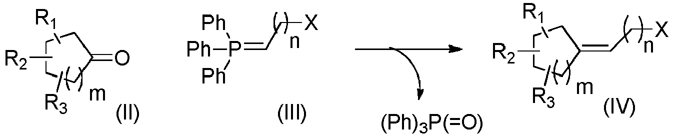
i) Anlagern eines Phosphorylids der Formel (III) an ein Cycloalkanon der Formel (II) gemäß einer Wittig-Reaktion,
wobei R1, R2 und R3 jeweils unabhängig ein Wasserstoffatom oder eine gesättigte oder ungesättigte, verzweigte oder unverzweigte C1-C5-Alkylgruppe darstellen;
m eine ganze Zahl zwischen 1 und 4 ist;
n eine ganze Zahl zwischen 2 und 4 ist;
der gesättigte Ring 5 bis 8 Kohlenstoffe umfasst, wobei die Gesamtzahl der Ringkohlenstoffe und der Reste R1, R2 und R3 zwischen 7 und 11 ist;
und X eine Nitril-, Carbonsäureester- oder Alkoholfunktion darstellt; und
ii) Umwandeln der Funktion X der erhaltenen Verbindung (IV) in Aldehyd durch Reduktion und/oder Oxidation.

10. Eine Verwendung mindestens einer Verbindung der Formel (I), wie in einem der Ansprüche 1 bis 5 definiert, als Duftmittel oder Duftstoffverbindung.

11. Verwendung mindestens einer Verbindung der Formel (I) gemäß einem der Ansprüche 1 bis 5 als geruchsüberdeckendes oder geruchsneutralisierendes Mittel.

12. Verwendung gemäß einem der Ansprüche 10 oder 11 von mindestens einer Verbindung der Formel (I) allein oder in Kombination mit mindestens einem weiteren Aroma- oder Duftstoffbestandteil und/oder mindestens einem Lösungsmittel und/oder mindestens einem Zusatzmittel.

13. Verwendung gemäß einem der Ansprüche 10 bis 12, um die organoleptischen Eigenschaften einer Substanz, einer Zusammensetzung oder eines Artikels zu verleihen, zu modifizieren oder zu verstärken.

14. Verwendung gemäß einem der Ansprüche 10 bis 13 zur Herstellung von Zusammensetzungen wie etwa Parfümzusammensetzungen, topischen Zusammensetzungen, insbesondere Kosmetikzusammensetzungen, oder auch Reinigungsmitteln.

## Claims

1. A compound of the following general formula (I): 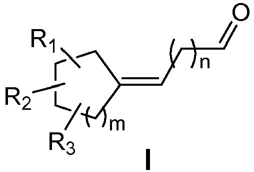 wherein:
- R1, R2 and R3 each independently represent a hydrogen atom or a saturated or unsaturated, branched or non-branched C1-C5 alkyl group;
- m is an integer between 1 and 4;
- n is an integer between 2 and 4;
**characterised in that** the ring is saturated and comprises from 5 to 8 carbons, that the total number of carbons of the ring and of the radicals R1, R2 and R3 is between 7 and 11
and it being understood that said compound of formula (I) is not:
- 6-cycloheptylidenehexanal
- 4-(4-methylcyclohexylidene)-butanal
- 4-(4-tert-butylcyclohexylidene)-butanal
- 4-(3,3,5-trimethylcyclohexylidene)-butanal
- 4-(3,3-dimethylcyclopentylidene)-butanal
- 4-(3-ethyl-3-methylcyclopentylidene)-butanal.

2. The compound according to claim 1, **characterised in that** m is equal to 1.

3. The compound according to claim 1 or 2, **characterised in that** n is equal to 4.

4. The compound according to claim 1 or 2, **characterised in that** n is equal to 2.

5. The compound according to claim 1, **characterised in that** it is chosen from 5-(2,4,4-trimethylcyclopentylidene)-pentanal, 6-(2,4,4-trimethylcyclopentylidene)-hexanal, 6-(2-methylcyclohexylidene)-hexanal, 6-(4-methylcyclohexylidene)-hexanal, 6-(4-tert-butylcyclohexylidene)-hexanal, 6-(4-tert-amylcyclohexylidene)-hexanal, 6-cyclooctylidenehexanal, 6-(3,3-dimethylcyclohexylidene)hexanal, 4-(2,4,4-trimethyl cyclopentylidene)butanal, 4-(2-pentylcyclopentylidene)-butanal, 4-(3,3-dimethylcyclohexylidene)-butanal, 5-(4,4-diethylcyclohexylidene)-pentanal and 5-cycloheptylidenepentanal.

6. A composition **characterised in that** it comprises at least one compound of general formula (I) as defined in claim 1 in the form of an isomer or a mixture of isomers, of an enantiomer or of a mixture of enantiomers, or of a racemic mixture, or of a diastereoisomer or of a mixture of diastereoisomers.

7. The composition according to the preceding claim, **characterised in that** it further comprises at least one other fragrancing substance.

8. A method of preparing a compound of formula (I) as described in claim 1 in which a cycloalkanone and a phosphorus ylide undergo a Wittig reaction, followed by a reduction and/or oxidation step depending on the ylide used previously.

9. A method according to claim 8 **characterized in that** it comprises the steps of: 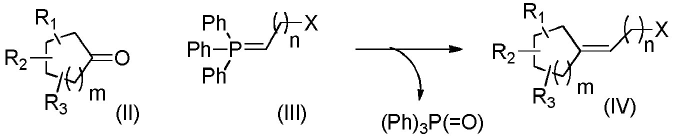
i) adding a phosphorus ylide of formula (III) onto a cycloalkanone of formula (II), according to a Wittig reaction
R1, R2 and R3 each independently representing a hydrogen atom or saturated or unsaturated, branched or non-branched C1-C5 alkyl group;
m being an integer between 1 and 4;
n being an integer between 2 and 4;
the ring being saturated, comprising from 5 to 8 carbons, the total number of carbons of the ring and of the radicals R1, R2 and R3 being between 7 and 11; and X representing a nitrile, carboxylic ester or alcohol function; and
ii) converting the function X of the compound (IV) obtained, into aldehyde, by reduction and/or oxidation.

10. The use of at least one compound of formula (I) as defined in one of claims 1 to 5 as a fragrant agent or compound.

11. The use of at least one compound of formula (I) as defined in one of claims 1 to 5 as an odour-masking or odour-neutralising agent.

12. The use, according to one of claims 10 or 11, of at least one compound of formula (I) alone or in combination with at least one other flavouring or perfuming ingredient, and/or at least one solvent, and/or at least one additive.

13. The use according to one of claims 10 to 12 to confer, modify or boost the organoleptic properties of a substance, a composition or an article.

14. The use according to one of claims 10 to 13 for the manufacture of compositions such as perfumery compositions, topical compositions, in particular cosmetics, or cleaning products.
